# FASCICULE DE BREVET EUROPEEN

(11) **EP 0 644 423 B1**
(45) Date de publication et mention de la délivrance du brevet: **15.12.1999**
(21) Numéro de dépôt: 94114425.5
(22) Date de dépôt: 14.09.1994
(51) Int. Cl.: G01N 33/487, G01N 33/49

(54) **Dispositif de mesure pour capteurs multizones amovibles**
Messvorrichtung für abnehmbare mehrzonige Sensoren
Measuring apparatus for detachable multizone sensors

(30) Priorité: 21.09.1993 FR 9311316
(43) Date de publication de la demande: 22.03.1995
(73) Titulaire: ASULAB S.A., CH-2501 Bienne (CH)
(72) Inventeur: Dinger, Rudolf, CH-2024 Saint-Aubin (CH); Hoffmann, Eric, CH-2563 Ipsach (CH)
(74) Mandataire: Patry, Didier Marcel Pierre

(56) Documents cités:
- WO-A-90/10861
- WO-A-92/21961

## Description

La présente invention concerne un dispositif de mesure destiné à être associé à un capteur amovible comportant une pluralité de zones actives à usage unique, ce capteur amovible étant nommé par la suite capteur multizone.

Plus particulièrement, la présente invention concerne un dispositif de coupe compris dans de tels dispositifs de mesure. Ce dispositif de coupe sert à couper, après utilisation d'une zone active pour effectuer une mesure, notamment une mesure du type électrochimique, un capteur multizone introduit dans le dispositif de mesure pour séparer la partie de ce capteur multizone comportant cette zone active utilisée du reste du capteur multizone.

Aux figures 1 et 2 est représenté un dispositif de mesure électrochimique tel que décrit dans la demande de brevet FR 93 01331.

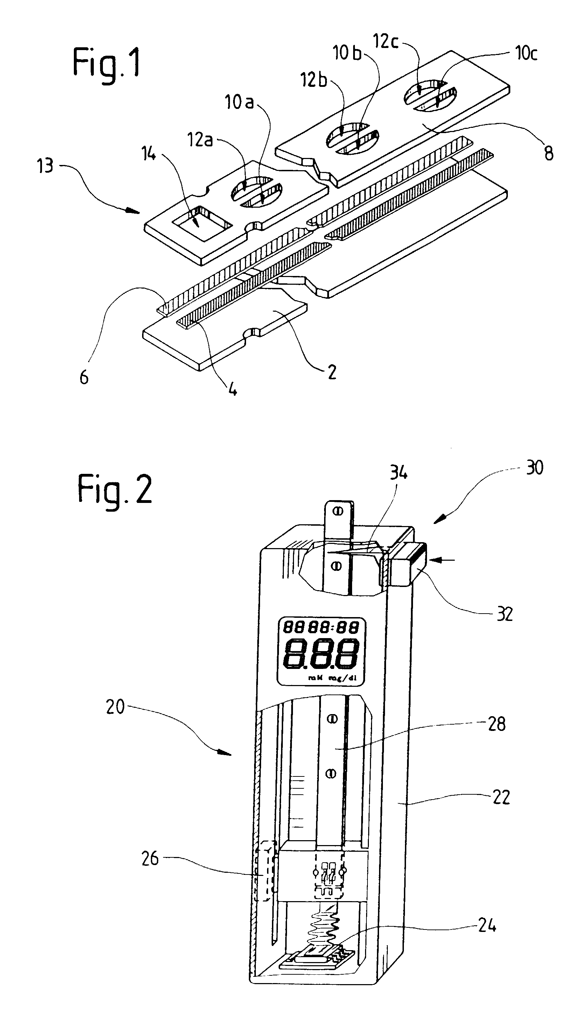

Sur la figure 1 est représenté partiellement et en perspective un éclaté d'un capteur multizone, comportant un substrat isolant 2 à la surface duquel sont prévues deux électrodes 4 et 6 servant à conduire un courant électrique de mesure. Sur ces électrodes 4 et 6 est prévu un revêtement 8 également isolant.

Le revêtement 8 comporte une première série d'ouvertures 10a, 10b, 10c au moins partiellement superposées à la première électrode 4 et une deuxième série d'ouvertures 12a, 12b, 12c superposées au moins partiellement à la deuxième électrode 6. A une extrémité 13 du revêtement 8 est prévue au moins une ouverture 14 servant à dégager une plage de contact électrique sur chacune des deux électrodes 4 et 6.

On notera que les électrodes 4 et 6 sont électriquement isolées l'une de l'autre et que la première série d'ouvertures 10a, 10b, 10c n'est pas superposée à la seconde électrode 6. De même, la deuxième série d'ouvertures 12a, 12b, 12c n'est pas superposée à la première électrode 4. Chaque paire d'ouvertures correspondante 10a et 12a, respectivement 10b et 12b, 10c et 12c définit une zone active du capteur multizone.

Le fonctionnement électrochimique d'un tel capteur multizone est sensiblement identique à celui d'un capteur monozone, tel que décrit par exemple dans la demande de brevet Wo 92/14836.

La substance à analyser, par exemple du sang dans une application possible à la mesure du glucose dans le sang, est apportée dans une des zones actives du capteur multizone. Pour effectuer une mesure électrochimique valable, il est nécessaire que seulement une des zones actives du capteur multizone soit recouverte par la substance à analyser, les zones actives restantes devant nécessairement être non utilisées.

Sur la figure 2 est représenté un dispositif de mesure électrochimique, désigné par la référence générale 20, destiné à recevoir un capteur multizone amovible du type de celui décrit à la figure 1.

Le dispositif de mesure électrochimique 20 comprend un boîtier 22 à l'intérieur duquel est prévue une électronique de mesure 24. En outre, ce dispositif de mesure électrochimique 20 comprend un dispositif d'avance 26 servant à déplacer le capteur multizone 28 introduit à l'intérieur du boîtier 22.

Afin de couper, après utilisation d'une zone active pour effectuer une mesure électrochimique, le capteur multizone 28 pour séparer la partie de ce capteur multizone 28, comportant cette zone active utilisée, du reste de celui-ci, il est prévu un dispositif de coupe 30.

Le dispositif de coupe 30 comprend un poussoir 32 et une lame 34 solidaire de ce poussoir 32. Le dispositif de coupe 30 est agencé de manière que le poussoir 32 est susceptible d'effectuer une course, selon une direction transversale au capteur multizone 28, suffisamment longue pour permettre à la lame 34 de traverser entièrement ce capteur multizone 28.

Le dispositif de coupe 30 décrit ci-avant présente divers inconvénients. Premièrement, il est nécessaire d'exercer une pression relativement élevée sur le poussoir 32 pour couper le capteur multizone 28. Deuxièmement, il est très difficile de doser manuellement la pression exercée sur le poussoir 32 étant donné la forte pression nécessaire à la coupe du capteur multizone 28. De ce fait, la partie sectionnée absorbe en fin de parcours de coupe une grande énergie de déformation dans un laps de temps très court, ce qui engendre un phénomène de détente propulsant la partie sectionnée relativement loin du boîtier 22. Ce dernier inconvénient est particulièrement désavantageux étant donné que le produit à analyser risque de s'éloigner au moins partiellement de la zone active dans laquelle il a été placé. De plus, la localisation de la partie sectionnée peut être rendue difficile étant donné la direction de propulsion indéterminée de la partie sectionnée. Lorsque le produit à analyser est un produit dangereux pour l'homme ou son environnement, ce dernier inconvénient rend l'utilisation du dispositif de mesure électrochimique décrit ci-avant peu recommandable.

Un but de la présente invention est de pallier les inconvénients mentionnés ci-dessus en fournissant un dispositif de mesure comportant un dispositif de coupe permettant un contrôle de l'avance de la lame de manière à assurer un petit éloignement de la partie sectionnée relativement au dispositif de mesure.

A cet effet, la présente invention concerne un dispositif de mesure, destiné à être associé à un capteur multizone amovible, comprenant un dispositif de coupe de ce capteur multizone amovible et caractérisé en ce que ce dispositif de coupe comporte un levier, une bielle et une lame, ledit levier effectuant une rotation autour d'un premier axe lorsqu'il est actionné, ladite bielle étant reliée mécaniquement audit levier et étant susceptible de subir une rotation autour d'un deuxième axe différent dudit premier axe et solidaire dudit levier. Ladite lame est reliée mécaniquement à ladite bielle et disposée à l'intérieur d'un guide définissant un trajet déterminé pour cette lame lorsque le levier est actionné. En outre, le dispositif de mesure est agencé de manière que la lame peut trancher le capteur multizone amovible mis en place dans ce dispositif de mesure lorsque le levier est actionné entre une position initiale de coupe et une position finale de coupe.

Un autre but de l'invention est de fournir un dispositif de coupe agencé de manière que la force de coupe exercée par la lame sur le capteur multizone amovible soit supérieure à la force exercée par l'utilisateur pour couper ce capteur multizone amovible placé dans le dispositif de mesure.

A cet effet, et selon des caractéristiques particulières du dispositif de mesure selon la présente invention, les premier et deuxième axes sont parallèles et le levier présente, dans un plan perpendiculaire à ces premier et deuxième axes, une dimension supérieure à la distance prévue entre ces premier et deuxième axes.

Le dispositif de mesure selon l'invention permet de transformer un mouvement circulaire du levier en un mouvement sensiblement rectiligne de la lame. De ce fait, il est possible de mieux contrôler la force exercée sur la lame. Ensuite, il résulte des caractéristiques particulières mentionnées ci-avant que la force de coupe exercée par la lame sur le capteur multizone est plus grande que la force exercée par un utilisateur sensiblement à l'extrémité libre du levier. Ainsi, la coupe du capteur multizone et le contrôle de la vitesse d'avance de la lame dans celui-ci sont facilités.

D'autres caractéristiques et avantages de l'invention seront décrits ci-après à l'aide de la description suivante faite en référence aux dessins annexés, donnés à titre d'exemples nullement limitatifs, dans lesquels :
- la figure 1, déjà décrite, montre en perspective un éclaté d'un capteur multizone amovible;
- la figure 2, déjà décrite, montre schématiquement un dispositif de mesure associé au capteur multizone amovible représenté à la figure 1;
- la figure 3 est une vue de dessus partiellement arrachée d'un dispositif de mesure selon l'invention, cette figure montrant un dispositif de coupe associé à un couvercle représenté dans une position initiale de coupe;
- la figure 4 est une vue en coupe de la figure 3 faite selon la ligne de coupe IV - IV;
- la figure 5 est une vue en coupe similaire à la figure 4, mais représente en traits tirés le couvercle dans une position intermédiaire de coupe et en trait continu ce couvercle dans une position finale de coupe;
- les figures 6 et 7 sont respectivement similaires aux figures 4 et 5, mais représentent une variante de réalisation du dispositif de coupe décrit aux figures 3 à 5.

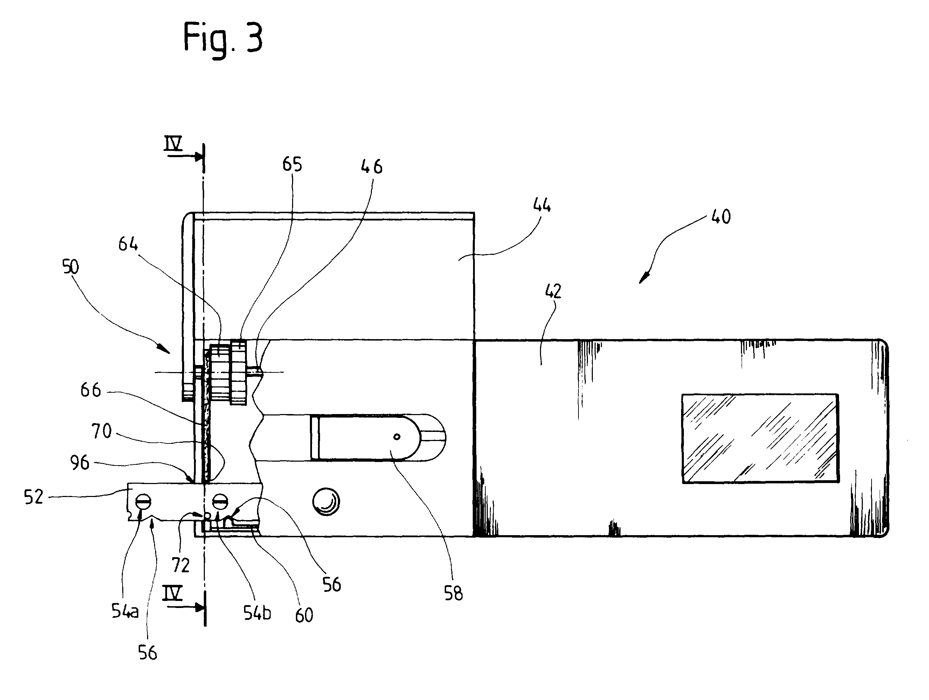

A l'aide des figures 3 à 5, on décrira ci-après un mode de réalisation d'un dispositif de mesure selon l'invention.

Sur la figure 3, le dispositif de mesure, désigné par la référence générale 40 comprend un boîtier 42 et un couvercle 44 susceptible de subir une rotation autour d'un premier axe de rotation 46. Le dispositif de mesure 40 comporte également un dispositif de coupe 50 servant à couper un capteur multizone amovible 52 associé au dispositif de mesure 40.

Le capteur multizone amovible 52 comporte une pluralité de zones actives 54a, 54b. Chacune des zones actives 54a, 54b du capteur multizone amovible 52 est à usage unique, c'est-à-dire qu'elle sert à effectuer une seule mesure d'une substance à analyser.

Après avoir utilisé une zone active pour effectuer une mesure, notamment une mesure du type électrochimique, il est prévu de couper le capteur multizone amovible 52 à l'aide du dispositif de coupe 50 pour séparer la partie de ce capteur 52 comportant cette zone active utilisée du reste de celui-ci.

Après avoir coupé le capteur multizone amovible , il est possible de déplacer ce capteur, a l'aide d'un dispositif d'avance (non-représenté) comprenant notamment un poussoir 58, pour permettre à une nouvelle zone active vierge 54a d'être située hors du boîtier 42. L'agencement du capteur 52 et du dispositif d'avance qui lui est associé permet qu'une seule zone active soit située hors du boîtier 42, cette zone active étant alors destinée à recevoir la substance à analyser lors d'une prochaine mesure.

Le capteur multizone amovible 52 peut être maintenu dans une pluralité de positions différentes à l'aide d'encoches de positionnement 56 coopérant avec un ressort de positionnement 60.

Le dispositif de coupe 50 comporte un levier constitué par le couvercle 44 solidaire d'un disque ou cylindre 64 dont l'axe central coïncide avec le premier axe de rotation 46. Le cylindre 64 peut être venu de formage avec le couvercle 44 ou être fixé à une pièce 65 faisant elle-même partie du couvercle 44 comme représenté sur les figures 3 à 5. Le dispositif de coupe 50 comprend en outre une bielle 66 reliée à une première extrémité au cylindre 64 de manière excentrique, cette bielle 66 étant susceptible de subir une rotation autour d'un deuxième axe 68 solidaire du couvercle 44. La bielle 66 est reliée solidairement à une lame 70 servant à trancher le capteur multizone amovible 52.

On notera que, de manière avantageuse, le capteur multizone amovible 52 comporte une pluralité d'ouvertures 72 (une seule ouverture étant représentée sur la figure 3), ces ouvertures 72 étant disposées le long du capteur 52. Chacune des ouvertures 72 est disposée de manière à être traversée par la lame 70 lors d'une coupe du capteur 52 maintenu dans l'une desdites positions différentes prévues pour ce capteur 52 dans le dispositif de mesure 40. Ces ouvertures 72 servent à absorber une énergie de déformation résultant de la pression exercée par la lame 70 sur la matière formant le capteur 52 lors d'une coupe de celui-ci.

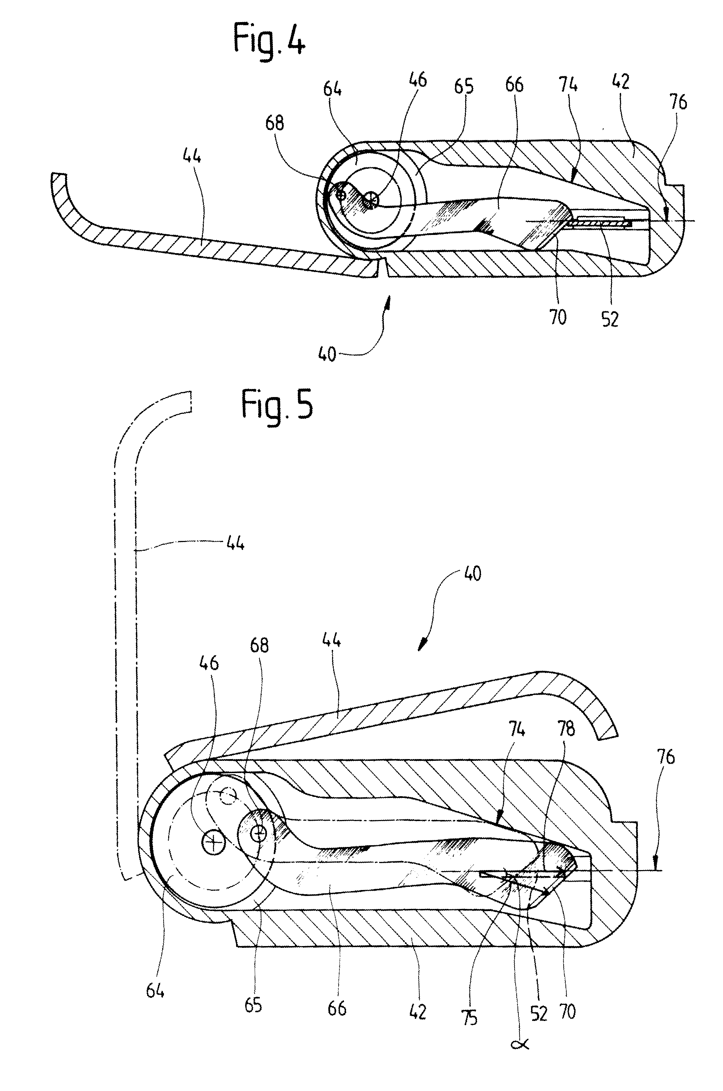

Sur les figures 3 et 4, le couvercle 44 et la lame 70 sont représentés dans une position initiale de coupe.

Sur la figure 5, le couvercle 44 et la lame 70 sont représentés en traits tirés dans une position intermédiaire de coupe et en trait plein dans une position finale de coupe. Lorsque le couvercle 44 est actionné entre ladite position initiale de coupe et ladite position finale de coupe, la lame 70 parcourt un trajet de coupe défini par un guide 74 ménagé à l'intérieur du boîtier 42.

On notera que le capteur multizone amovible 52 est de forme allongée et plate définissant un plan général 76 de ce capteur 52. Lors de l'actionnement du couvercle 44 entre la position initiale de coupe et la position finale de coupe, le capteur 52 est tranché par la lame 70 selon une direction de coupe 78.

Le guide 74 est agencé de telle manière que la lame 70 parcourt un trajet entre sa position initiale de coupe et sa position finale de coupe définissant une direction d'avance 75 de cette lame 70 décalée angulairement d'un angle α relativement à la direction de coupe 78. De ce fait, la pénétration de la lame dans la matière est facilitée. En effet, lors du tranchement du capteur 52, la lame 70 subit un mouvement perpendiculaire au plan général 78 du capteur multizone amovible 52. Il découle également de ce fait une usure uniforme de la lame 70 et par conséquent une usure plus faible que dans le cas où la zone de contact entre la lame 70 et le capteur 52 reste identique tout au long de la coupe de ce capteur 52.

On remarquera encore que les premier et deuxième axes de rotation 46 et 68 sont parallèles entre eux et que la distance séparant ces deux axes est inférieure à la longueur du levier formé par le couvercle 44. L'agencement du dispositif de coupe 50 transforme un mouvement circulaire du couvercle 44 actionné par un utilisateur en un mouvement sensiblement rectiligne de la lame 70. Cet agencement présente l'avantage de transformer un mouvement circulaire uniforme, c'est-à-dire à vitesse angulaire constante, en un mouvement rectiligne de la lame 70 dont la vitesse varie de manière sinusoïdale entre ladite position initiale de coupe et ladite position finale de coupe. De ce fait, la vitesse de la lame 70 lors de l'attaque du capteur 52 et en fin de coupe du capteur 52 est très faible.

La forme de la bielle 66 est particulièrement bien adaptée à un dispositif de mesure présentant une hauteur relativement faible. Cependant, lorsque le couvercle 44 est actionné entre ladite position initiale de coupe et ladite position finale de coupe, la bielle 66 intercepte le premier axe de rotation 46. De ce fait, dans ce mode de réalisation particulier, l'axe 46 relié au cylindre 64, sur lequel est fixé l'axe 68 servant également au maintien de la bielle 66, ne doit pas faire saillie hors du cylindre 64 dans la région destinée à la bielle 66.

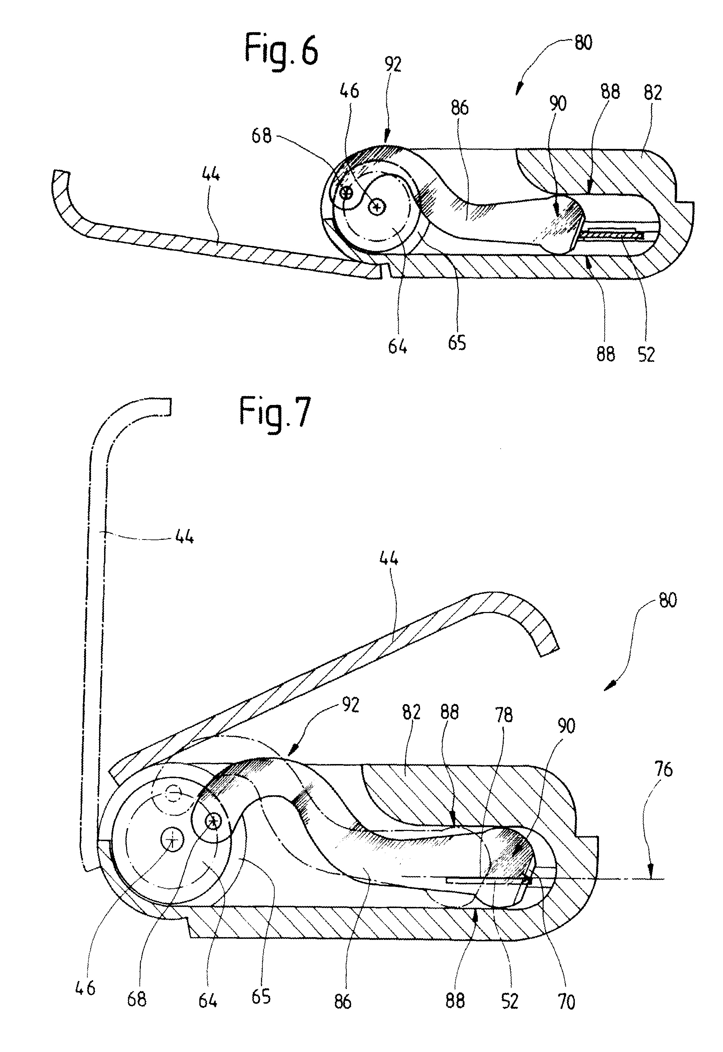

Sur les figures 6 et 7 est représenté une variante du mode de réalisation décrit à l'aide des figures 3 à 5.

Dans cette variante, le dispositif de mesure 80 comporte également un boîtier 82 et un couvercle 44 servant de levier d'actionnement d'une bielle 86 reliée solidairement à une lame 70. Le système d'actionnement de la lame 70 solidaire de la bielle 86 au moyen du couvercle 44 est identique au système décrit ci-avant.

Cette variante diffère essentiellement du mode de réalisation décrit ci-avant par la forme de la bielle 86 et par l'agencement du guide 88 définissant un trajet de coupe pour la lame 70.

Le guide 88 définit un trajet rectiligne pour la lame 70 entre une position initiale de coupe représentée à la figure 6 et une position finale de coupe représentée en trait plein à la figure 7. Sur cette figure 7 est également représentée une position intermédiaire de coupe en traits tirés.

Bien que le trajet imposé par le guide 88 à la tête 90 de la bielle 86 portant la lame 70 soit rectiligne et parallèle à la direction de coupe 78, la lame 70 subit un léger mouvement vertical, c'est-à-dire perpendiculaire au plan général 76 du capteur 52 lorsque le couvercle 44 et la lame 70 sont actionnés par un utilisateur entre ladite position initiale de coupe et ladite position finale de coupe. Ce léger mouvement vertical résulte d'une légère rotation de la tête 90 de la bielle 86 qui présente une forme circulaire.

La bielle 86 présente dans cette variante un coude 92. Ce coude 92 est formé de telle manière que la bielle 86 n'intercepte jamais le premier axe 46 autour duquel le couvercle 44 est susceptible de subir une rotation. Dans ce cas-ci, l'axe 46 peut sans autre faire saillie hors du cylindre 64 du côté de la bielle 86. En particulier, cet axe 46 peut traverser entièrement la région prévue pour la bielle 86 de manière à être fixé au couvercle 44 de l'autre côté du cylindre 64 relativement à la bielle 86.

On remarquera que le couvercle 44 est susceptible de subir une rotation d'un angle d'environ 180° dans le mode de réalisation et dans la variante de ce mode de réalisation décrits ci-avant. Cependant, il est sans autre possible de prévoir pour le couvercle 44 un chemin angulaire d'un angle différent. On notera aussi que le couvercle 44 peut également servir à protéger l'orifice 96 (représenté à la figure 3) ménagé dans le boîtier 42 ou 82 pour l'introduction du capteur 52 à l'intérieur de ce boîtier.

Finalement, on remarquera qu'il est possible de concevoir des modes de réalisation de l'invention dans lesquels la lame 70 est susceptible de subir un mouvement relativement à la bielle 66, 86 à laquelle elle est reliée mécaniquement.

## Revendications

1. Dispositif de mesure, destiné à être associé à un capteur multizone amovible (52), comprenant un dispositif de coupe (50) dudit capteur multizone amovible et caractérisé en ce que ledit dispositif de coupe comporte un levier (44), une bielle (66; 86) et une lame (70), ledit levier effectuant une rotation autour d'un premier axe (46) lorsqu'il est actionné, ladite bielle étant reliée mécaniquement audit levier et susceptible de subir une rotation autour d'un deuxième axe (68) différent dudit premier axe et solidaire dudit levier, ladite lame étant reliée mécaniquement à ladite bielle et disposée à l'intérieur d'un guide (74; 88) définissant un trajet déterminé pour cette lame lorsque ledit levier est actionné, ledit dispositif de mesure étant agencé de manière que ladite lame permet de couper ledit capteur multizone amovible placé dans ledit dispositif de mesure lorsque ledit levier est actionné entre une position initiale de coupe et une position finale de coupe.

2. Dispositif de mesure selon la revendication 1, caractérisé en ce que ledit deuxième axe (68) est parallèle audit premier axe (46), ledit levier (44) ayant, dans un plan perpendiculaire audit premier et deuxième axes, une dimension supérieure à la distance prévue entre ces premier et deuxième axes.

3. Dispositif de mesure selon la revendication 1, caractérisé en ce que ladite lame (70) est reliée rigidement à ladite bielle (66; 86).

4. Dispositif de mesure selon la revendication 1, caractérisé en ce que ledit trajet défini par ledit guide (74; 88) pour ladite lame (70) est sensiblement rectiligne.

5. Dispositif de mesure selon la revendication 1, destiné à être associé à un capteur multizone amovible (52) de forme allongée et sensiblement plate définissant un plan général (76) de ce capteur multizone amovible et une direction de coupe (78) pour ladite lame (70) dans ce plan général, ce dispositif de mesure étant caractérisé en ce que ledit guide (74) est agencé de manière que, lorsque ledit capteur multizone amovible (52) est placé dans ledit dispositif de mesure, ledit trajet défini par ledit guide entre une position initiale de coupe de la lame (70) et une position finale de coupe de cette lame, correspondant respectivement auxdites position initiale de coupe et position finale de coupe dudit levier, détermine une direction d'avance (75) de ladite lame décalée angulairement relativement à ladite direction de coupe (78).

6. Dispositif de mesure selon l'une quelconque des revendications précédentes, caractérisé en ce que ladite bielle (86) présente un coude (92) agencé de manière que cette bielle n'intercepte jamais ledit premier axe (46).

7. Dispositif de mesure selon l'une quelconque des revendications précédentes, caractérisé en ce que ledit levier (44) est constitué par un couvercle servant en outre à protéger un poussoir (58) servant à l'avance dudit capteur multizone amovible (52).

8. Dispositif de mesure selon la revendication 7, caractérisé en ce que ledit couvercle (44) sert également à fermer un orifice (96) servant à l'introduction du capteur multizone amovible (52) à l'intérieur dudit dispositif de mesure.

9. Dispositif de mesure selon l'une quelconque des revendications précédentes, caractérisé en ce que ledit levier (44) peut subir une rotation d'un angle sensiblement égale à 180°.

10. Dispositif de mesure selon l'une quelconque des revendications précédentes, caractérisé en ce que ce dispositif de mesure et ledit capteur multizone amovible (52) auquel il est associé sont agencés pour mesurer le taux de glucose dans le sang.

## Patentansprüche

1. Meßvorrichtung, die dazu vorgesehen ist, einem entnehmbaren Mehrzonen-Sensor (72) zugeordnet zu werden, eine Schneidvorrichtung (50) für den entnehmbaren Mehrzonen-Sensor enthält und dadurch gekennzeichnet ist, daß die Schneidvorrichtung einen Hebel (44), eine Stange (66; 86) und eine Klinge (70) umfaßt, der Hebel, wenn er betätigt wird, eine Drehung um eine erste Achse (46) ausführt, die Stange mit dem Hebel mechanisch verbunden ist und eine Drehung um eine zweite Achse (68), die von der ersten Achse verschieden und mit dem Hebel fest verbunden ist, ausführen kann und die Klinge mit der Stange mechanisch verbunden ist und in einer Führung (74; 88) angeordnet ist, die, wenn der Hebel betätigt wird, eine bestimmte Bahn für diese Klinge definiert, wobei die Meßvorrichtung so beschaffen ist, daß die Klinge das Schneiden des in der Meßvorrichtung angeordneten entnehmbaren Mehrzonen-Sensors ermöglicht, wenn der Hebel zwischen einer Schneid-Anfangsposition und einer Schneid-Endposition betätigt wird.

2. Meßvorrichtung nach Anspruch 1, dadurch gekennzeichnet, daß die zweite Achse (68) zur ersten Achse (46) parallel ist, wobei der Hebel (44) in einer zu den ersten und zweiten Achsen senkrechten Ebene eine Abmessung besitzt, die größer als der zwischen diesen ersten und zweiten Achsen vorgesehene Abstand ist.

3. Meßvorrichtung nach Anspruch 1, dadurch gekennzeichnet, daß die Klinge (70) starr mit der Stange (66; 86) verbunden ist.

4. Meßvorrichtung nach Anspruch 1, dadurch gekennzeichnet, daß die durch die Führung (74; 88) für die Klinge (70) definierte Bahn im wesentlichen geradlinig ist.

5. Meßvorrichtung nach Anspruch 1, die dazu vorgesehen ist, einem entnehmbaren Mehrzonen-Sensor (52) mit länglicher und im wesentlichen ebener Form zugeordnet zu werden, wobei die Form eine allgemeine Ebene (76) dieses entnehmbaren Mehrzonen-Sensors und eine Schneidrichtung (78) für die Klinge (70) in dieser allgemeinen Ebene definiert, wobei diese Meßvorrichtung dadurch gekennzeichnet ist, daß die Führung (74) so beschaffen ist, daß, wenn der entnehmbare Mehrzonen-Sensor (52) in der Meßvorrichtung angeordnet ist, die Bahn, die durch die Führung zwischen einer Schneid-Anfangsposition der Klinge (70) und einer Schneid-Endposition dieser Klinge, die der Schneid-Anfangsposition bzw. der Schneid-Endposition des Hebels entsprechen, definiert ist, eine Vorschubrichtung (75) der Klinge bestimmt, die in bezug auf die Schneidrichtung (78) in Winkelrichtung verschoben ist.

6. Messvorrichtung nach irgendeinem der vorangehenden Ansprüche, dadurch gekennzeichnet, daß die Stange (86) eine Biegung (92) aufweist, die so beschaffen ist, daß diese Stange niemals die erste Achse (46) stört.

7. Messvorrichtung nach irgendeinem der vorangehenden Ansprüche, dadurch gekennzeichnet, daß der Hebel (44) durch einen Deckel gebildet ist, der außerdem dazu dient, eine dem Vorschub des entnehmbaren Mehrzonen-Sensors (52) dienende Schubstange (58) zu schützen.

8. Meßvorrichtung nach Anspruch 7, dadurch gekennzeichnet, daß der Deckel (44) außerdem dazu dient, eine Öffnung (96) zu verschließen, die der Einführung des entnehmbaren Mehrzonen-Sensors (52) in den Innenraum der Meßvorrichtung dient.

9. Meßvorrichtung nach irgendeinem der vorangehenden Ansprüche, dadurch gekennzeichnet, daß der Hebel (44) eine Drehung um einen Winkel von im wesentlichen gleich 180° ausführen kann.

10. Meßvorrichtung nach irgendeinem der vorangehenden Ansprüche, dadurch gekennzeichnet, daß diese Meßvorrichtung und der entnehmbare Mehrzonen-Sensor (52), dem sie zugeordnet ist, so beschaffen sind, daß sie den Gehalt an Glucose im Blut messen.

## Claims

1. Measurement arrangement, intended to be associated with a multiple zone removable sensor (52) comprising a cutting arrangement (50) for said multiple zone removable sensor and characterized in that said cutting arrangement includes a lever (44), a crank (66; 86) and a blade (70), said lever performing a rotation around a first axle (46) whenever it is actuated, said crank being mechanically coupled to said lever and adapted to undergo a rotation around a second axle (68) different from said first axle and fixed to said lever, said blade being mechanically coupled to said crank and arranged in the interior of a guide (74; 88) defining a predetermined path for such blade whenever said lever is actuated, said measurement arrangement being arranged in a manner such that said blade enables the cutting of said multiple zone removable sensor placed within said measurement arrangement whenever said lever is actuated between an initial cutting position and a final cutting position.

2. Measurement arrangement according to claim 1, characterized in that said second axle (68) is parallel to said first axle (46), said lever (44) having a dimension greater than the distance provided between said first and second axles in a plane perpendicular to said first and second axles.

3. Measurement arrangement according to claim 1, characterized in that said blade (70) is rigidly coupled to said crank (66; 86).

4. Measurement arrangement according to claim 1, characterized in that said path defined by said guide (74; 88) for said blade is substantially rectilinear.

5. Measurement arrangement according to claim 1, intended to be associated with a multiple zone removable sensor (52) of elongated form and substantially flat defining a general plane (76) for such multiple zone removable sensor and a cutting direction (78) for said blade (70) in such general plane, such measurement arrangement being characterized in that said guide (74) is arranged in a manner such that, when said multiple zone removable sensor (52) is placed in said measurement arrangement, said path defined by said guide between an initial cutting position of the blade (70) and a final cutting position of said blade, corresponding respectively to said initial cutting position and final cutting position of said lever, determines a travel direction (75) for said blade angularly offset relative to said cutting direction (78).

6. Measurement arrangement according to any one of the preceding claims, characterized in that said crank (86) exhibits an elbow (92) arranged in a manner such that said crank never intercepts said first axle (46).

7. Measurement arrangement according to any one of the preceding claims, characterized in that said lever (44) is constituted by a cover serving in addition to protect a push-piece (58) serving for the advancing of said multiple zone removable sensor (52).

8. Measurement arrangement according to claim 7, characterized in that said cover (44) also serves to close an orifice (96) serving for the introduction of the multiple zone removable sensor (52) into the interior of measurement arrangement.

9. Measurement arrangement according to any one of the preceding claims, characterized in that said lever (44) can undergo a rotation through an angle substantially equal to 180°.

10. Measurement arrangement according to any one of the preceding claims, characterized in that such measurement arrangement and said multiple zone removable sensor (52) with which it is associated are arranged to measure the proportion of glucose in the blood.
